# EUROPEAN PATENT APPLICATION

(11) **EP 3 906 946 A1**
(43) Date of publication of application: **10.11.2021**
(21) Application number: 20172714.6
(22) Date of filing: 04.05.2020
(51) Int. Cl.: A61K 47/69

(54) **ANTIPATHOGEN VESICLE**

(71) Applicant: EK Biosciences GmbH, 4312 Magden (CH)
(72) Inventor: The designation of the inventor has not yet been filed
(74) Representative: Vossius & Partner Patentanwälte Rechtsanwälte mbB

(57) **Abstract**

The present invention relates to a vesicle, wherein the vesicle displays on its surface two or more different binding sites to one or more different docking domains on a pathogen.

## Description

The present invention relates to a vesicle, wherein the vesicle displays on its surface two or more different binding sites to one or more different docking domains on a pathogen.

A wide range of natural and (semi)-synthetic carrier systems that are capable to administer - mostly as payload - therapeutically active agents to specific compartments in the body have been disclosed in patent and scientific literature. For example, aerosols, fluidic uni- and multilamellar liposomes (Rudokas et al. 2016; Ghatage et al. 2017), more rigid cross-linked liposomes (Chen et al. 1997), elastin-like polypeptide particles (Osborne et. al. 2008), and hybrid elastin-like polypeptide/liposome nanoparticles (Zhang et al. 2018) have been described or disclosed.

Surface functionalization of vesicles plays an important role to cope with specific requirements posed upon by biological systems as well as physiological and transport factors. Functionalization strategies for liposomes used in targeted cancer therapies have been developed that result in lower side effects / higher biocompatibility and improved targeting properties (Riaz et al. 2018) as well as in improved uptake in macrophages (Weber et. al. 2019).

Various formulation issues of liposome delivery systems for inhalation have been addressed with a focus on treatment of lung cancer (Rudokas et al. 2016). A method using polymerized surface modified liposomes for oral and/or mucosal delivery of vaccines, allergens and therapeutics targeted to a specific site or cell type has also been disclosed (Chen and Lou 1997).

Various types of vesicles and nanoparticles are potentially suitable as carriers for targeted or non targeted transport of pharmaceutically active ingredients (PAI) to the pulmonary systems (Ong et al. 2014), among others as natural or surface modified liposomes (Rudokas et al. 2016), micelles (Pellosi et al. 2018), elastin-like recombinamers (polypeptides) (Ibáñez-Fonseca et al. 2019), polymeric nanoparticles (d'Angelo et al. 2015), and spray-dried powders (Rezazadeh et.al. 2018). With respect to lipid based vesicles synthetic, semi-synthetic or naturally-occurring lipids have been claimed. Cationic, anionic as well as zwitterionic lipid vesicles are known to be used in carrier processes. Liposomes used as carrier systems have been in part surface modified using different classes of proteins among others to control clearance in the target compartments (Torchilin et al. 1981).

Vesicles applied as aerosols to treat pulmonary diseases, lung infections, and lung cancer in practice are so far primarily applied as non targeted carriers of pharmaceutically active ingredients (PAI) as payload and lack the efficient targeting to e.g. inflammatory or cancerous tissue (Rudokas et al. 2016). Serious limitation of pulmonary delivery of non targeted carriers like e.g. the short residence time in the lung have to be overcome. Results obtained on the pulmonary pharmacokinetics of e.g. Paclitaxel administered by aerosols or intravenously demonstrate the superior long-term efficacy of the aerosol administration (Rudokas et al. 2016).

In order to more selectively reach defined lung compartments like trachea, bronchi, bronchioles, and alveoli vesicle size are - preferably unimodal size selected in the range of 10 nm to 10 µm, preferably in the range of 0.5 to 5 µm (Smyth et al. 2011; Rudokas et al. 2016; Lee et al. 2018).

Weers (Weers 2006) teaches a system for treating or providing prophylaxis against a pulmonary infection comprising a mixture of free antiinfectives and antiinfectives encapsulated in a lipid-based composition.

Galili and Ogawa (Galili and Ogawa 2016) disclose a novel treatment of selected animals and humans infected with influenza virus in early stages of the disease with C-gal/SA modified liposomes in order to decrease the infection period and decrease further complications by this disease. The treatment is based on the ability of influenza virus to bind to SA epitopes on cells and on the binding of macrophages to the Fc part of the natural anti-Gal antibody (the most abundant natural antibody in humans). Medications based on liposome inhalation are commercially available, e.g. Arikayce, an inhaled antibiotic taken with a nebulizer (Ehsan et al. 2015; Zhang et al. 2018; Kaufman, 2019; Shirley 2019).

In a recent study, mixed polymeric micelles based on tocopheryl succinate-polyethylene glycol 1000 and 5000 Da (TPGS1K and TPGS5K) were synthesized and loaded with Paclitaxel (PTX) to treat lung cancer (Rezazadeh et al. 2018).

In the last years the application of biopolymer-based elastin or elastin-derived molecules have been investigated in drug delivery systems due to their specific properties such as elasticity, self-assembly e.g. on liposomes, long-term stability and biological activity. It has been shown that the application of elastin-like polypeptides (ELPs) as drug carriers do not cause any inflammation at the site of injection. They are degradable by proteolysis which reduces their adverse side effect due to long-term accumulation in the body. In addition, the ELP's proteinaceous structure allows for easy genetic coupling of peptide ligands to them for targeting to the respective receptors (Kübler and Albrecht 2018; Albrecht and Kübler 2019).

Depending on properties of compounds and dose to be administered numerous different commercial inhalators are available, e.g. systems for manual jet vaporization / nebulization, electrically / electronically / charge based nebulization, (ultra)sound vaporization (e.g. Scheuch et al. 2001; Gardenhire et al. 2017). Especially in the context of asthma and COPD treatment using inhalator systems have widely been applied, tested and valuated, among others.

A method of reducing the loss of antiinfectives encapsulated in a lipid-based composition upon nebulization comprising administering an aerosolized pharmaceutical formulation comprising a mixture of free antiinfective and antiinfective encapsulated in a lipid-based composition has been claimed (Weers 2006).

A method of treating bronchoalveolar carcinoma, carcinomatosis with lymphangitic spread or primary and metastatic lung cancers by administering one or more bioactive agents by inhalation of a lipid composition. Bioactive agents, however, were focused on cancer treatment and preferably comprise cisplatin, carboplatin or a taxane (Perez-Soler and Pilkiewicz 2002).

Methods to treat chronic Pseudomonas aeruginosa infection in cystic fibrosis via inhalation of Amikacin antibiotic loaded liposomes have been published (Caimmi et al. 2018; Bilton et al. 2019).

A method to administer Calcifediol-loaded liposomes for local treatment of pulmonary bacterial infections has been published (Castoldi 2017).

A method to administer antibiotics in liposomes for tuberculosis therapy by inhalation of liposomes has been published (Justo and Moraes 2003).

A method to treat chronic lung infection with Pseudomonas aeruginosa using ciprofloxacin loaded liposomes (Haworth et al. 2019).

A method for improvement of tolerance for therapeutically effective agents delivered by inhalation comprising a pretreatment of a patient with a nebulized lidocaine or a lidocaine-like compound administered immediately or up to about thirty minutes before administration of the primary therapeutically effective agent has been disclosed (Hofmann 2005). However, vesicles combining the docking and compound releasing processes were not described in this disclosure.

A method to administer antiinfectives (such as antibiotics) encapsulated in lipid-based particles to treat or ameliorate pulmonary infection in a cystic fibrosis patient to reduce antibiotics application or dosing frequency has been disclosed (Boni et al. 2005).

Studies have demonstrated the safety of liposomes for pulmonary administration. Animal models showed, e.g. that inhalation of HSPC (hydrogenated soy phosphatidylcholine) liposomes caused no pathological effects on alveolar macrophages (Myers et al. 1993). Studies performed with human volunteers have established the safety of liposomes for inhalation (Canonico et al. 1994; Myers et al. 1993; Waldrep et al. 1994; Jendle et al. 1995; Gilbert et al. 1997; Khanna et al. 1997; Parthasarathy and Gilbert 1999; Skubitz and Anderson 2000; Ten et al. 2002; Verschraegen et al. 2004; Corcoran et al. 2006; Monforte et al. 2013).

Biomolecular interaction principles have been investigated on an experimental as well as computational level and applied to address and to interact with specific sites of molecular receptors as well as of pathogens. Depending on the nature of the targets probes typically are selected from the group of mono- or polyclonal antibodies, antibody fragments, proteins, nucleic acids, as well as other natural or synthetic molecular structures like aptamers able to interact based on polar, nonpolar, ionic / electrostatic interaction forces, or formation of chemical bonds.
About ten years ago a special approach using so called targeted covalent inhibitors (TCI) has been established in drug discovery and development to address and subsequently inactivate low binding sites: An inhibitor bearing an additional bond-forming functional group of low reactivity that, following binding to the target protein, is positioned to react rapidly with a specific non-catalytic residue close to the target site (Singh et al. 2011). As - in contrast to the use of classical inhibitors - this modification is in essence irreversible it has been coined "warhead"; several classes of TCI reactants are known in the meanwhile to react among others with cysteine, lysine, and methionine (Gehringer et al. 2019; Li et al. 2019; Rowlands et al. 2019). Recently a dual warhead application has been disclosed (Chen et al. 2017).

There are few studies evaluating various agents as therapy for MERS-CoV in animal models. In the rhesus macaques model, interferon-α2b-ribavirin combination decreased viral replication within 8 h of MERS-CoV infection. In a primate model, the mortality rate at 36 h post-inoculation was reduced from 67% in untreated to 0-33% in animals treated with a combination of interferon-plb and either lopinavir or ritonavir (Al-Tawfiq and Memish 2017).

There are many coronaviruses in wild animal populations, but only a few developed the ability to infect humans, comprising SARS-CoV, MERS-CoV and the newly emerging SARS-CoV-2. All of them cause severe respiratory, gastrointestinal, and central nervous system diseases. Coronaviruses are capable of adapting to new environments through mutation and recombination with relative ease and hence are programmed to alter host range and tissue tropism efficiently, e.g. SARS-CoV-2 which is in close phylogenetic relationship to RaTG13 (96.2 %) shows evidence for a bat origin while a putative intermediate host is currently unclear (Fang 2016). Corona virus exhibit glycoprotein "spikes", large protrusions from the virus surface, giving coronaviruses the appearance of having crowns. In addition to mediating virus entry, the spike is a critical determinant of viral host range and tissue tropism and a major inducer of host immune responses (Fang 2016). The coronavirus spikes contain two segments, the N-terminal S1 and the C-terminal S2. While S1 consists of a N-terminal domain (S1-NTD) and a C-terminal domain (S1-CTD) and facilitates the interaction of the virus with the host receptor, the S2 segment consists of a fusion peptide, two conserved heptad repeats (HR), a transmembrane domain and a short intracellular tail and induces a conformational change leading to the fusion of the virus with the host cellular membrane, followed by the release of the viral genome into the cytoplasm of the host cell (Alsaadi et al. 2019). Important for the release of the viral genome is the separation of S1 and S2, which is mediated by a host protease. For SARS-CoV spike, mutagenesis, structural and lipid mixing studies have suggested a motif SFIEDLLFNKVTLADAGF which is conserved across the coronavirus family implicating that our described approach could also be functional for treatment of other coronavirus caused diseases occurring in the future. The spike S1-CTD of SARS-CoV binds the cellular receptor zinc peptidase Angiotensin-Converting Enzyme 2 (ACE2), which is also a target of SARS-CoV-2. In addition to the ACE2-binding domain, SARS-CoV-2 contains a K403R change in its spike S1-NTD compared to the SARS-CoV spike, forming an adjacent RGD motif at the interaction surface. This RGD motif provides an additional binding site of SARS-CoV-2 with the host cells' integrins (Yan et al. 2020).

It has been shown that for the MERS-CoV an analogue of the heptad repeat (HR) 2 binds to HR1 to form a fusion core to bring the virus into a close position for fusion to the cell membrane. Peptides derived from the HR2 region, such as HR2P can also interact with the HR1 domain in the viral S protein to form heterogenous six-helix bundles therefore blocking the viral fusion with the host cell membranes. When applied intranasally, viral titers in lungs of mice challenged with MERS-CoV could be reduced >1000-fold; mice treated before the challenge were highly protected from MERS-CoV infection (Lu et al. 2014).

A heptad repeat (HR) 2 analogue with improved pharmaceutical property,HR2P-M2, has been shown highly effective against MERS-CoV infection in vitro and in vivo when applied intranasally in mice by potently blocking MERS-CoV S protein-mediated membrane fusion (Channappanavar et al. 2015).

ACE2 is a membrane anchored glycoprotein that functions as a zinc-binding carboxypeptidase and has been implicated, among others, in the regulation of heart function. It is also as a functional receptor for SARS-CoV. ACE2 is also expressed in various organs such as the kidneys, liver, lungs and on blood vessels. The extracellular part of ACE2 contains a membrane-distal zinc metallopeptidase domain and a membrane-proximal collectrin-like domain (Towler et al. 2004). The major activity of ACE2 lies in the conversion of Angiotensin II to Angiotensin 1-7. While Angiotensin 1-7 is a blood-vessel dilator, Angiotensin II, among its many other known biological effects, is a potent vasoconstrictor and promotes renal sodium retention both of which lead to hypertension (Wysocki et al. 2010). Both SARS-CoV and SARS-CoV-2 use ACE2 as a cell receptor to enter the host cells (Lu et al. 2020), thereby blocking the receptor. Blocking the ACE2 receptor leads to the accumulation of Angiotensin II and therefore to an increased blood pressure and hypertension (Wysocki et al. 2010).

The use of ACE2 for treatment of sepsis via reduction of AngII accumulation that in consequence reduces inflammation has been disclosed; application / transport of formulated ACE2 protein or ACE2-encoding nucleic acid (e.g. incorporated in a vector) in e.g. lipids (Loibner et al. 2008 & 2017).

ACE2 fused to the Fc portion of immunoglobulin has just been reported to neutralize SARS-CoV-2 in vitro (Lei et al. 2020).

The soluble human recombinant ACE2 (rACE2) lacks the transmembrane domain and circulates in small amounts in the blood; it is able to convert Angiotensin II to Angiotensin 1-7 and prevent the increase of blood pressure (Batlle et al. 2020; Wysocki et al. 2010). Upon Angiotensin II infusion, Angiotensin II levels can be lowered while Angiotensin 1-7 levels can be increased in the blood of mice when rACE2 was added. rACE2 has only a partial effect on kidney Angiotensin II levels despite complete normalization of plasma Angiotensin II levels. This shows that only the amount of Angiotensin II that was circulating could be degraded by rACE2 present in serum. The missing anchoring site of the soluble form of ACE2, contributing to retain the native form of ACE2 on the cell plasma membrane may explain, in part, why neither kidney nor heart ACE2 activity were increased with different doses of rACE2 whereas serum ACE2 activity increased markedly (Wysocki et al. 2010). Although rACE2 addition could convert Angiotensin II and prevent blood pressure, it has a short residence time, with a calculated half-life *in vivo* of only 8.5 hours (Wysocki et al. 2010). Therefore, increasing the ACE2 activity at the tissue level should have important effects that cannot be achieved by rACE2 and vice versa.

The present invention relates to means and methods to overcome the disadvantages of low affinity binding of single chemical compounds / therapeutics to pathogens, the short residence times of the therapeutics in the respiratory system and the single functionality of the therapeutics.

In particular, the present invention relates to the following items.
1. A vesicle, wherein the vesicle displays on its surface two or more different binding sites to one or more different docking domains on a pathogen.
2. The vesicle of item 1, wherein at least one of the two or more binding sites on the surface of the vesicle comprise(s) a linker between the vesicle surface and the binding site.
3. The vesicle of item 2, wherein the linker is a polypeptide, a polysaccharide, or an organic polymer.
4. The vesicle of item 3, wherein the organic polymer is polyethylene glycol (PEG), preferably comprising 1 to 200 monomers.
5. The vesicle of any one of the preceding items, wherein one of the two or more different binding sites to one or more different docking domains on the pathogen is an integrin or lectine.
6. The vesicle of any one of the preceding items, wherein one of the two or more different binding sites to one or more different docking domains on the pathogen is angiotensin converting enzyme 2 (ACE2).
7. The vesicle of any one of the preceding items, wherein one of the two or more different binding sites to one or more different docking domains on the pathogen is a chemical warhead for covalent binding to a reactive residue of a docking domain on the surface of the pathogen, preferably wherein the reactive residue is part of a phospholipid, polysaccharide and/or extracellular polypeptide, in particular an amino acid thereof like asparagine, threonine, lysine, tyrosine, cysteine, histidine, arginine
8. The vesicle of item 7, wherein the warhead and at least one other binding site of the two or more different binding sites to one or more different docking domains on the pathogen are arranged on the vesicle in a spatial proximity to allow covalent binding of the vesicle to at least one of the one or more different docking domains on the pathogen.
9. The vesicle of any one of the preceding items, wherein the vesicle further displays a digestive enzyme, in particular an enzyme with polypeptide digestive activity, in particular a serine protease.
10. The vesicle of any one of the preceding items, wherein the vesicle further displays a macrophage binding site, in particular an activating domain, in particular an Fc domain of an IgG antibody.
11. The vesicle of any one of the preceding items, wherein the vesicle has a diameter between 0.05 and 200 µm.
12. An aerosol comprising the vesicle of any one of the preceding items.
13. The vesicle of any one of the preceding items or the aerosol of item 12 for use in medicine.
14. The vesicle of any one of the preceding items or the aerosol of item 12 or 13, which binds to a pathogen.
15. The vesicle or aerosol of item 14, further comprising a payload, in particular an anti-sense oligonucleotide to block the pathogen DNA / RNA, a ribonuclease or trypsin or an antiviral drug or viricid.
16. The vesicle or aerosol of item 14 or 15, wherein the pathogen is a virus.
17. The vesicle or aerosol of item 16 for use in treating a viral infection.
18. The vesicle or aerosol of item 16 or 17, wherein the virus is selected from the group consisting of Rhinovirus, RSV, HIV, Influenza virus, Parainfluenza virus, Metapneumovirus, Coronavirus, Enterovirus, Adenovirus, Bocavirus, Polyomavirus, Herpes simplex virus, and Cytomegalovirus.
19. The vesicle or aerosol of item 14, wherein the pathogen is a bacterium or fungus.
20. The vesicle or aerosol of item 18, wherein the Coronavirus is of the genus α-CoV, β-CoV, γ-CoV or δ-CoV.
21. The vesicle or aerosol of item 18, wherein the Coronavirus is selected from the group consisting of Human coronavirus OC43 (HCoV-OC43), Human coronavirus HKU1 (HCoV- HKU1), Human coronavirus 229E (HCoV-229E), Human coronavirus NL63 (HCoV-NL63, New Haven coronavirus), Middle East respiratory syndrome-related coronavirus (MERS-CoV or "novel coronavirus 2012"), Severe acute respiratory syndrome coronavirus (SARS-CoV or "SARS-classic"), and Severe acute respiratory syndrome coronavirus 2 (SARS-CoV-2 or "novel coronavirus 2019").
22. The vesicle of any one of items 13 to 21, wherein the vesicle is to be administered intravenously.
23. The aerosol of any one of items 13 to 21, wherein the aerosol is to be administered by inhalation or orally.
24. A solid surface coated with the vesicle of any one of items 1 to 11.
25. The solid surface of item 24, which is a mask or an air filter.
26. A method for diagnosing a disease, the method comprising the use of the vesicle of any one items 1 to 11 or of the aerosol of item 12, wherein the vesicle or aerosol comprises a detectable label.

Accordingly, in a first embodiment, the invention relates to a vesicle, wherein the vesicle displays on its surface two or more different binding sites to one or more different docking domains on a pathogen.

Within the present invention, a vesicle may comprise targeted liposomes, elastin-like coated structures, micelles, inorganic and/or polymer based nanoparticles.

One or more, in particular two, of the at least two binding sites may be an antibody, antibody fragment, a nanobody, an antigen, a cell membrane receptor, a protein A, a protein C, or an artificial recognition element, wherein the binding site has affinity for the pathogen. In some embodiments, two of the at least two binding sites may be an antibody, antibody fragment, a nanobody, an antigen, cell membrane receptor, a protein A, a protein C or an artificial recognition element. One or more, in particular two, of the at least two binding sites may in some embodiments of the invention be a chemical ligand, such as, but not limited to, a small molecule ligand, a peptide, an aptamer, an oligonucleotide,
Within the present invention, the terms "antibody fragment", "antigen", or "an artificial recognition element" are understood as binding sites having specific binding affinity to the pathogen, but that not necessarily comprise all of the elements of an antibody.

Similar or higher affinities of pathogens to vesicles as compared to pathogens to tissue interaction as well as the presence of high concentrations of potential binding sites for the pathogens on the vesicles will immediately and permanently reduce viral load and thus reduce cause of inflammation and allow reconstruction of healthy mucus. Higher affinities of the interaction partners are achieved by e.g. multiple site attachment by fostering an interaction of multiple docking domains of the pathogens with the corresponding binding sites displayed on the surface of the vesicles, and/or by formation of a covalent bond between the pathogen and the vesicle.

In some embodiments of the present invention, the vesicle displays on its surface two or more different binding sites to one or more different docking domains on a pathogen, wherein at least two binding sites bind to the same docking domain on the pathogen.

In some embodiments of the present invention, the vesicle displays on its surface two or more different binding sites to one docking domain on a pathogen.

In some embodiments of the present invention, the vesicle displays on its surface two or more different binding sites to two or more different docking domains on a pathogen, wherein at least two of the two or more different binding sites bind to different docking domains on the pathogen.

As such, within the present invention, multiple binding sites (e.g. ACE2 receptors, or other pathogen docking sites of cells) are attached to the surface of a vesicle allowing multiple interactions with the pathogen surface. The multiple binding sites can either be chemically identical or chemically different.

It has also been found by the inventors that the therapeutic application of small molecules to the respiratory system does not allow to selectively target certain specific areas / compartments in the airways or the lung, whereas surprisingly the size of the vesicle as such or the sieze of a vesicle formulated as aerosol according to the invention can be adjusted in order to cover all or just particular areas of the respiratory system. Furthermore, it has been surprisingly found that specific binding of the vesicles of the invention to a pathogen leads to a reduction of available docking domains on the surface of the pathogen for entry into targeted cells of the host. It is preferred within the present invention that the binding affinity of the vesicle to the pathogen is higher than the binding affinity of the docking domains on the surface of the vesicle to cells of the host organism.

A further benefit of the invention is that the therapeutic application of small molecules to the respiratory system fulfills a single task, binding to the target cells to cause a therapeutic effect. The multipurpose vesicle of the invention, in particular the vesicle in the form of an aerosol, enables e.g. the neutralization of a pathogen by multiple mechanisms and initiates the degradation of the pathogen loaded vesicles, in particular delivered as an aerosol, by activation of the immune system. In some embodiments, the vesicle or in particular the vesicle formulated as aerosol, may carry a pharmaceutically active ingredient to be delivered to the pathogen itself or the pathogen residing area.

A further benefit of the invention is that liposomes with functionalized surfaces, such as those known from the prior art, rely on the selective non-covalent interaction of a binding group with the pathogen. The combined approach of the present invention allows selective capturing and fixing. In particular, within the present invention, one of the at least two binding sites may trigger a non-covalent interaction to selectively dock onto the intended target pathogen, whereas another binding sites of the at least two different binding sites may form a covalent essentiallynon-reversible chemical bond to fix the vesicle to the pathogen. Different binding sites may be envisaged to form a covalent bond to a docking domain on a pathogen. For example, within the present invention, a warhead may be used to form a covalent bond.

Within the present invention, the binding site(s) may be in close proximity to the vesicle surface or may be detached from the vesicle surface. In one embodiment, the invention thus relates to a vesicle, wherein at least one of the two or more binding sites on the surface of the vesicle comprise(s) a linker between the vesicle surface and the binding site.

The linker may result in an improved accessibility of the binding site(s) and/or direct a secondary binding site into the proximity of the first binding site and/or improve accessibility of the binding site to two different docking domains. Within the present invention, the chemical nature of the linker is not particularly limited as long as it results in increasing the space between the vesicle surface and the binding site while retaining or preferably improving the binding ability of the binding site to the pathogen. As such, it is preferred that with the linker the binding site retains flexibility. It is also preferred that with the linker sequence the binding site, in case of having a secondary and/or tertiary structure, maintains its native conformation. Accordingly, within the present invention, the linker may, inter alia, be a polypeptide, polysaccharide or an organic polymer. In a particular embodiment of the invention, the organic polymer may be a polyethylene glycol (PEG), preferably a PEG comprising 1 to 200 monomers.

In one embodiment of the invention, one of the two or more different binding sites to one or more different docking domains on the pathogen may be an integrin or lectine. Integrins are transmembrane receptors that facilitate cell-extracellular matrix (ECM) adhesion. Upon ligand binding, integrins activate signal transduction pathways that mediate cellular signals such as regulation of the cell cycle, organization of the intracellular cytoskeleton, and movement of new receptors to the cell membrane. The presence of integrins allows rapid and flexible responses to events at the cell surface (e.g. signal platelets to initiate an interaction with coagulation factors). Several types of integrins exist, and within the present invention, one vesicle may have multiple different types on its surface.

Within the present invention, one of the two or more different binding sites to one or more different docking domains on the pathogen may be an angiotensin converting enzyme 2 (ACE2). Accordingly, the vesicle of the present invention may comprise on its surface an integrin and/or ACE2 in order to bind to a pathogen. In general, Angiotensin-converting enzyme 2 (ACE2) is an enzyme attached to the outer surface (cell membranes) of cells in the lungs, arteries, heart, kidney, and intestines. ACE2 also serves as the entry point into cells for some coronaviruses, as it is known from the prior art. However, ACE2 has never been used to direct virus particles away from cells of the human body. Accordingly, the vesicles of the present invention may comprise on their surface ACE2 molecules, preferably ACE2 molecules exhibiting an increased binding affinity to virus particles as compared to native ACE2. Such increased affinity may be achieved by introducing mutations into the natural ACE2 sequence, in particular into the binding site to viral particles. Whereas viral particles naturally use ACE2 as entry point into cells, ACE2 on the surface of the vesicles of the invention may be used to trap and fix viral particles without enabling their entry into the vesicle. Therefore, binding affinity of a mutant ACE2 to the viral particle may be enhanced to native ACE2, because the ability of the virus to detach from ACE2 inside the cell may be abolished.

In one embodiment, the invention relates to the vesicle of the invention wherein one of the two or more different binding sites to one or more different docking domains on the pathogen is a chemical warhead for covalent binding to the pathogen, particularly to its cysteine, lysine, aspartic acid or other reactive amino acid residue(s). That is, the second of the two binding sites may contain a warhead, a low molecular weight site that might form stable interactions with reactive residues of a docking domain on the surface of a pathogen, for example phospholipids, polysaccharides and/or extracellular polypeptides, in particular amino acids thereof like asparagine, threonine, lysine, tyrosine, cysteine, histidine, arginine.

In some embodiments of the invention, the vesicle may contain one or more payloads such as anti-sense oligonucleotides to block the pathogen DNA / RNA, ribonuclease or trypsin or antivirals or viricids.

In a further embodiment, the vesicle of the invention further displays a digestive enzyme, in particular an enzyme with polypeptide digestive activity. Such digestive activity may digest polypeptides on the surface of the viral particle, in particular polypeptides essential to the viral particle to enter host cells. A digestive enzyme within the present invention may, for example, be a serine protease. Such a digestive enzyme may, for example, cleave a polypeptide exposed on the surface of the pathogen used for entry into host cells. In a particular embodiment, the digestive enzyme cleaves the spike protein on the surface of a coronavirus and thus prevents binding of the spike protein to a host cell.

The vesicle may further display a macrophage binding site, in particular activating domain, in particular an Fc domain of an IgG antibody. It is thus envisaged within the present invention that at least one of the at least two different binding sites of the vesicle directs the vesicle to a pathogen, whereas the macrophage activating domain, in particular the Fc domain of an IgG antibody, directs macrophages to the complex of vesicle and pathogen.

In one embodiment, the vesicle of the present invention may have a diameter between 0.05 and 200 µm, in particular 0.1 to 100 µm, more particularly 0.1 to 20 µm, even more particularly 0.1 to 10 µm.

In a further embodiment, the invention relates to an aerosol comprising the vesicle of the invention. Within the present invention, an aerosol may be, but is not limited to, a suspension or emulsion of vesicles or liquid droplets containing vesicles in air or another gas. Accordingly, the invention relates to an aerosol comprising the vesicle. In some embodiments, the aerosol may comprise a mixture of vesicles and solvent. In further embodiments, the vesicle and/or aerosol may be formulated as suspension or emulsion. The aerosol particles have diameters typically less than 10 µm, preferably 0.5 to 5 µm

In another embodiment, the invention relates to medical uses of the means provided herein. Accordingly, the invention relates to, inter alia, the vesicle or the aerosol of the invention disclosed herein in medicine. In a particular embodiment of the invention the vesicle and/or the aerosol of the invention bind to a pathogen. In a further embodiment, the pathogen is a virus.

In a further embodiment, the invention relates to the vesicle, in particular the aerosol containing the vesicle, of the invention, or a pharmaceutical formulation comprising the vesicle and/or aerosol of the invention, for use in treating a viral infection. Within the present invention, the viral infection may be of any kind. It is however preferred that the viral infection is caused by a virus selected from the group consisting of Rhinovirus, RSV, Influenza virus, Parainfluenza virus, Metapneumovirus, Coronavirus, Enterovirus, Adenovirus, Bocavirus, Polyomavirus, Human Immunodeficiency Virus (HIV), Herpes simplex virus, and Cytomegalovirus. Within the present invention, the Coronavirus may in particular be of the genus α-CoV, β-CoV, γ-CoV or δ-CoV. More particularly, the Coronavirus may be selected from the group consisting of Human coronavirus OC43 (HCoV-OC43), Human coronavirus HKU1 (HCoV- HKU1), Human coronavirus 229E (HCoV-229E), Human coronavirus NL63 (HCoV-NL63, New Haven coronavirus), Middle East respiratory syndrome-related coronavirus (MERS-CoV or "novel coronavirus 2012"), Severe acute respiratory syndrome coronavirus (SARS-CoV or "SARS-classic"), and Severe acute respiratory syndrome coronavirus 2 (SARS-CoV-2 or "novel coronavirus 2019").

Within the present invention, the vesicle may be administered according to the preference of the skilled person. However, it is preferred that the vesicle is to be administered intravenously. The aerosol of the invention, however, is preferably administered by inhalation or orally.

In a further embodiment, the invention relates to a solid surface coated with the vesicle of the invention. The solid surface may, for example, be a mask, in particular the part of the mask covering the mouth of the user of the mask. In a further embodiment, the solid surface is an air filter, for example an air filter to remove pathogens in a medical device or air conditioning system.

In a further embodiment, the invention relates to a method for diagnosing a disease, the method comprising the use of the vesicle of the invention, in particular the aerosol of the invention comprising the vesicle, wherein the vesicle or aerosol comprises a detectable label. The detectable label may be a fluorescent label. As such, the vesicle or the aerosol comprising the vesicle may be administered to the airway for diagnostic purposes, e.g. bronchoscopy.

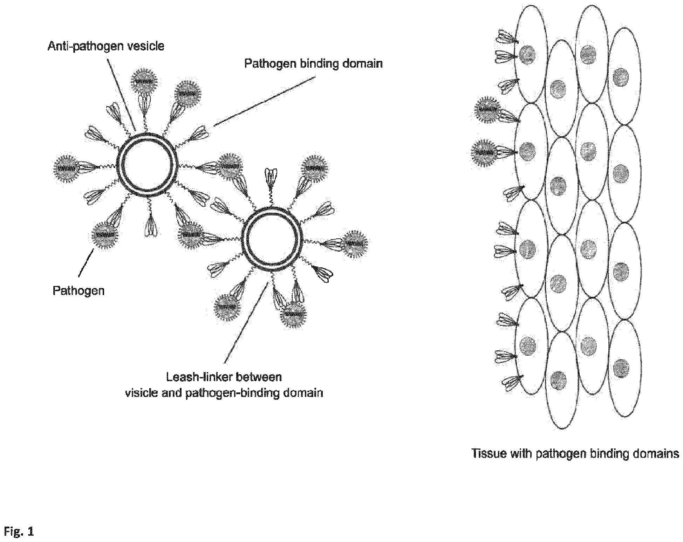

Fig.1 shows the vesicle of the invention, wherein the binding sites are shown with or without linker (leash).

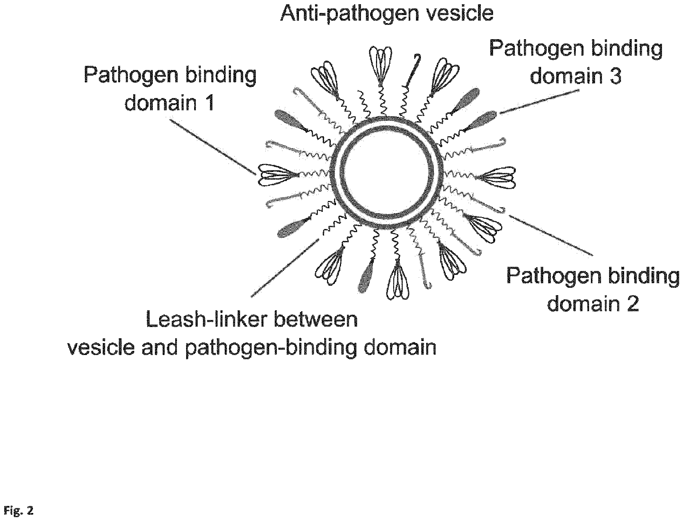

Fig. 2 shows an embodiment of the invention wherein two or more different binding sites such as ACE2 receptors and integrins, are on the vesicle in close proximity to each other providing different interaction options for the pathogen and thus further strengthening the affinity of the inhibition.

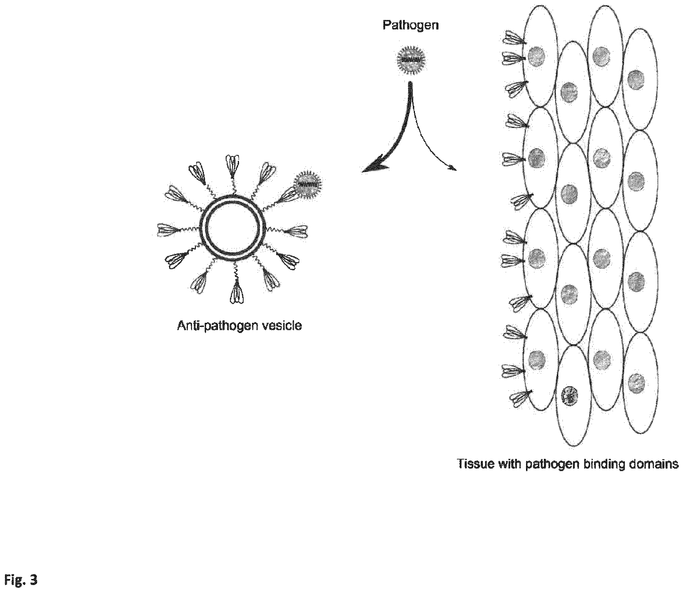

Fig. 3 depicts a possible mechanism of the vesicles of the invention, without being bound by theory.

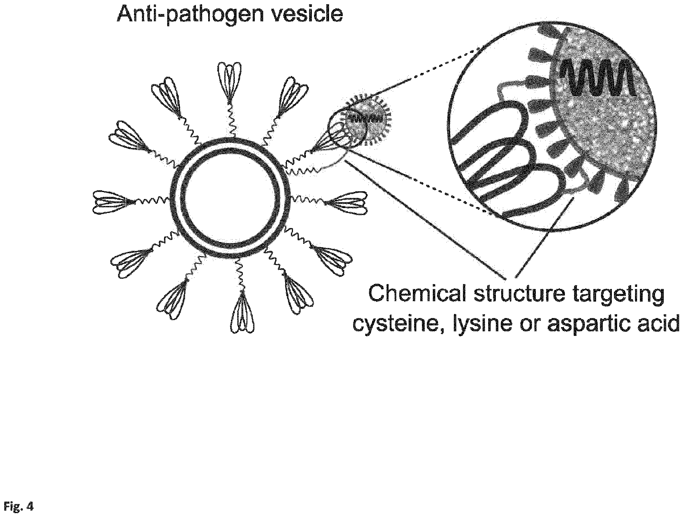

Fig. 4 shows an embodiment of the invention wherein the vesicle is presenting a receptor inhibitor such as ACE2 and a molecule containing chemical structures targeting amino acids such as a cysteine, lysine or aspartic acid, such as a warhead, to create a covalent non-reversible linkage between the vesicle and the pathogen after selective binding to the receptor inhibitor.

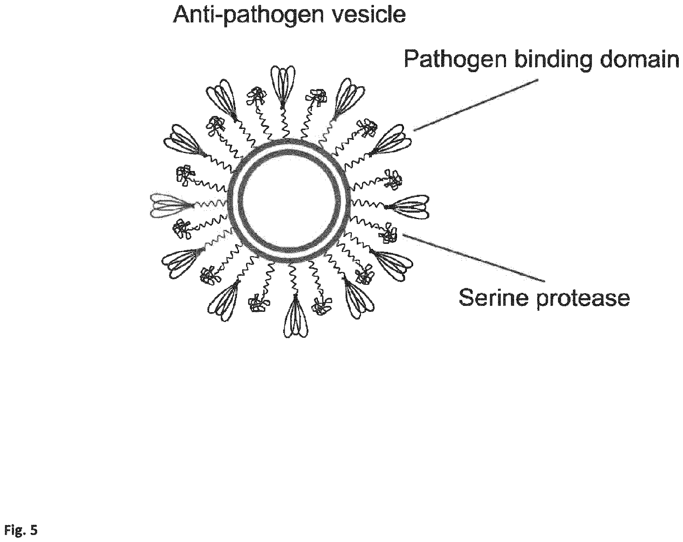

Fig. 5 shows an embodiment of the invention wherein the vesicle presents a selective pathogen binding group, e.g. ACE2 and an enzyme (e.g. a serine protease) to partly digest the pathogen (e.g. the peptide link between the S1 and S2 spike) after binding to the vesicle.

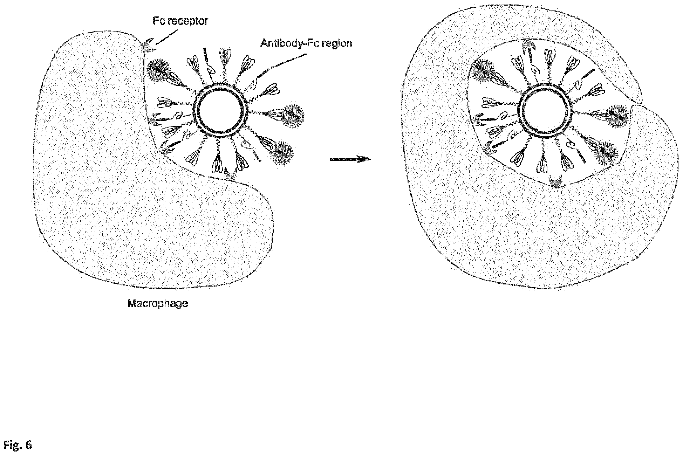

Fig. 6 shows an embodiment of the invention wherein the vesicle presents a selective pathogen surface binding group, e.g. ACE2 and a macrophage activating / binding domain, e.g. the Fc part of a human IgG antibody.

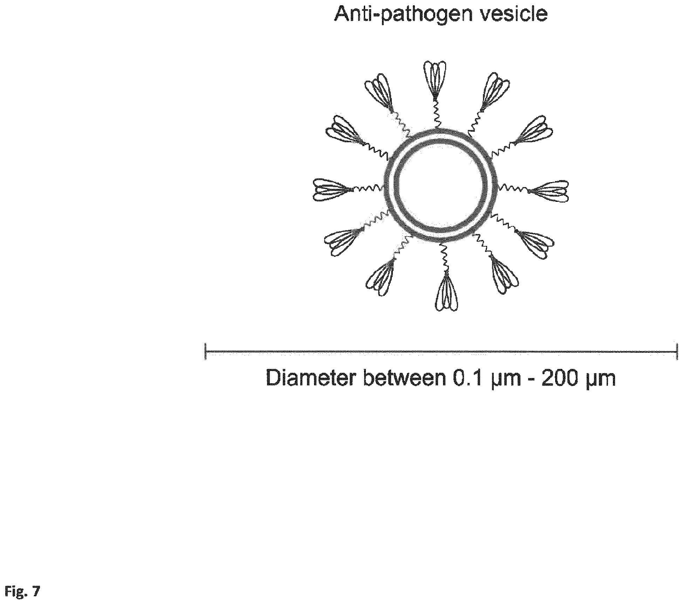

Fig. 7 shows an embodiment of the invention wherein the vesicle of any of the above embodiments with diameters selected between 0.5 and 200 µm targets specific parts of the respiratory system.
Fig. 8 shows an embodiment of the invention wherein the vesicle presents pathogen receptor sites triggering the fusion of the pathogen with the vesicle, the vesicle containing one or more payloads such as anti-sense oligonucleotides to block the pathogen DNA / RNA, ribonuclease or trypsin or antiviral drugs or viricids.

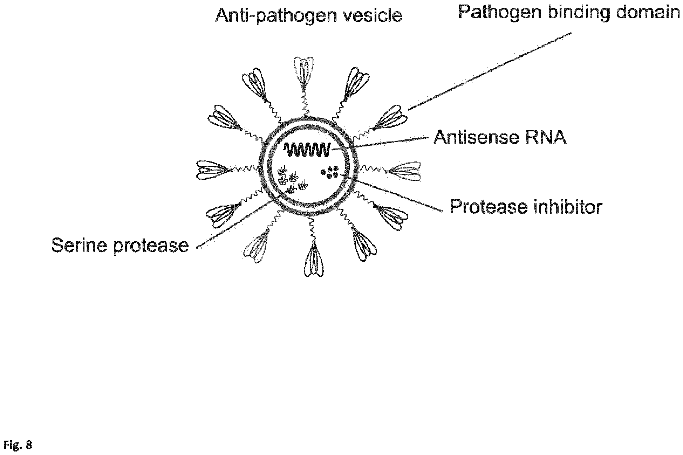

Fig. 8 shows an embodiment of the invention wherein the vesicle in a size range of 1 to 200 µm is applied as aerosols to humans and animals.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

The general methods and techniques described herein may be performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990).

While aspects of the invention are illustrated and described in detail in the drawings and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below. The invention also covers all further features shown in the figures individually, although they may not have been described in the previous or following description. Also, single alternatives of the embodiments described in the figures and the description and single alternatives of features thereof can be disclaimed from the subject matter of the other aspect of the invention.

Furthermore, in the claims the word "comprising" does not exclude other elements or steps, and the indefinite article "a" or "an" does not exclude a plurality. A single unit may fulfill the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

Aspects of the present invention are additionally described by way of the following illustrative non-limiting examples that provide a better understanding of embodiments of the present invention and of its many advantages. The following examples are included to demonstrate preferred embodiments of the invention. It should be appreciated by those of skill in the art that the techniques disclosed in the examples which follow represent techniques used in the present invention to function well in the practice of the invention, and thus can be considered to constitute preferred modes for its practice. However, those of skill in the art should appreciate, in light of the present disclosure that many changes can be made in the specific embodiments which are disclosed and still obtain a like or similar result without departing from the spirit and scope of the invention. A number of documents including patent applications, manufacturer's manuals and scientific publications are cited herein. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

### LITERATURE

d'Angelo I, Conte C, Miro A, Quaglia F, Ungaro F, Pulmonary drug delivery: a role for polymeric nanoparticles. Curr Top Med Chem 2015, 15:386-400
Albrecht H and Kübler E. Systematic Meta-Analysis Identifies Co-Expressed Kinases and GPCRs in Ovarian Cancer Tissues Revealing a Potential for Targeted Kinase Inhibitor Delivery. Pharmaceutics 2019, 11:454.
Al-Tawfiq JA and Memish ZA, Update on therapeutic options for Middle East Respiratory Syndrome Coronavirus (MERS-CoV), Exp Rev Anti-Infective Ther 2017, 15:269-275
Alsaadi EAJ, Neuman BW and Jones IM. A Fusion Peptide in the Spike Protein of MERS Coronavirus. Viruses 2019, 11: E825.
Batlle D, Wysocki J and Satchell K. Soluble angiotensin-converting enzyme 2: a potential approach for coronavirus infection therapy? Clin Sci (Lond) 2020, 134:543-545.
Bilton D, Pressler T, Fajac I, Clancy JP, Sands D, Minic P, Cipolli M, Galeva I, Sole A, Quittner AL, Liu K, McGinnis JP 2nd, Eagle G, Gupta R, Konstan MW, CLEAR-108 Study Group. Amikacin liposome inhalation suspension for chronic Pseudomonas aeruginosa infection in cystic fibrosis, J Cystic Fibrosis, 2019, S1569-1993(19):30833-1.
Boni L, Miller BS 2016, Sustained release of antiinfectives. US20160271125
Caimmi D, Martocq N, Trioleyre D, Guinet C, Godreuil S, Daniel T, Chiron R, Positive effects of liposomal Amikacin for inhalation on Mycobacterim abcessus in cystic fibrosis patients, Open Forum Infect Dis 2018, 17;5(3):ofy034.
Canonico AE, Plitman JD, Conary JT, et al. No lung toxicity after repeated aerosol or intravenous delivery of plasmid-cationic liposome complexes. J Appl Physiol 1994, 77:415-419.
Castoldi A, Herr C, Niederstraßer J, Labouta HI, Melero A, Gordon S, Schneider-Daum N, Bals R, Lehr CM, Calcifediol-loaded liposomes for local treatment of pulmonary bacterial infections, Eur J Pharm Biopharm 2017, 118:62-67
Channappanavar R, Lu L, Xia S, Du L, Meyerholz DK, Perlman S, Jiang S, Protective Effect of Intranasal Regimens Containing Peptidic Middle East Respiratory Syndrome Coronavirus Fusion Inhibitor Against MERS-CoV Infection, J Infectious Diseases 2015, 212:1894-1903
Chen Y, Lou Y. 2017, US10253029B2 Dual warhead covalent inhibitors of FGFR-4.
Chen H, Langer RS 1997, US006060082A Polymerized liposomes targeted to M cells for oral or mucosal drug delivery.
Corcoran TE, Venkataramanan R, Mihelc KM, et al. Aerosol deposition of lipid complex amphotericin-B (Abelcet) in lung transplant recipients. Am J Transplant 2006, 6: 2765-2773.
Ehsan Z and Clancy JP. Management of Pseudomonas aeruginosa infection in cystic fibrosis patients using inhaled antibiotics with a focus on nebulized liposomal amikacin. Future Microbiol 2015, 10:1901-1912.
Fang L. Structure, Function, and Evolution of Coronavirus Spike Proteins. Annu Rev Virol 2016,3: 237-261.
Galili U, Ogawa H, US20160256388A1 Composition and methods for treatment of resporatory tract infections
Gardenhire DS, Burnett D, PhD, Strickland S, Myers TR, A guide to aerosol delivery devices for respiratory therapists, 4th Edition, American Association for Respiratory Care 2017
Gehringer M, Laufer SA, Emerging and Re-Emerging Warheads for Targeted Covalent Inhibitors: Applications in Medicinal Chemistry and Chemical Biology, J Med Chem 2019, 62:5673-5724.
Ghatage T, Jadhav S, Kore V. Review on Stealth Liposomes: Novel Drug Delivery System 2017, IJSETR 6, 2744-2750
Gilbert BE, Knight C, Alvarez FG, et al. Tolerance of volunteers to cyclosporine a-dilauroylphosphatidylcholine liposome aerosol. Am J Respir Crit Care Med 1997, 156:1789-1793.
Haworth CS, Bilton D, Chalmers JD, Davis AM, Froehlich J, Gonda I, Thompson B, Wanner A, O'Donnell AE, Inhaled liposomal ciprofloxacin in patients with non-cystic fibrosis and chronic lung infection with Pseudomonas aeruginosa (ORBIT-3 and ORBIT-4): two phase 3, randomised controlled trials, Lancet Respir Med 2019, 7:213-226.
Hofmann T, Montgomery AB, Stapleton K, Baker WR 2005, US7452524B2 Method for improvement of tolerance for therapeutically effective agents delivered by inhalation.
Ibáñez-Fonseca A, Flora T, Acosta S, Rodriguez-Cabello JC, Trends in the design and use of elastin-like recombinamers as biomaterials, Matrix Biology 2019, 84, 111-126
Jendle J, Karlberg BE, Persliden J, et al. Delivery and retention of an insulin aerosol produced by a new jet nebulizer. J Aerosol Med 1995 8: 243-254.
Justo OR and Moraes AM, Incorporation of Antibiotics in Liposomes Designed for Tuberculosis Therapy by Inhalation, Drug Delivery 2003, 10:201-207.
Kaufman MB. Pharmaceutical Approval Update. P T. 2019 44:42-44.
Khanna C, Anderson PM, Hasz DE, et al. Interleukin-2 liposome inhalation therapy is safe and effective for dogs with spontaneous pulmonary metastases. Cancer 1997, 79:1409-1421.
Kübler E and Albrecht H. Large set data mining reveals overexpressed GPCRs in prostate and breast cancer: potential for active targeting with engineered anti-cancer nanomedicines. Oncotarget 2018, 9:24882-24897.
Lee H, Zhang D, Laskin DL, Jin Y, Functional Evidence o fPulmonary Extracellular Vesicles in Infectious and Noninfectious Lung Inflammation. J Immunology 2018, 201:1500-1509.
Lei C, Fu W, Qian K, Li T, Zhang S, Ding M, Hu S, Potent neutralization of 2019 novel coronavirus by recombinant ACE2-Ig, bioRxiv preprint 2020, doi: https://doi.org/l0.1 101/2020.02.01.929976
Li B, Rong D, Wang X, Targeting Protein-Protein Interaction with Covalent Small-Molecule Inhibitors. Current Topics in Medicinal Chemistry 2019, 19:1872-1876.
Loibner H, Schuster M, Treatment of inflammatory illnesses with ACE2, 2008, US 9,561.263 B2
Loibner H, Schuster M, Treatment of inflammatory illnesses with ACE2, 2017, US20180117126A1
Lu L, Liu Q, Zhu Y, Chan KH, Qin L, Li Y, Wang Q, Chan JFW, Du L, Yu F, Ma C, Ye S, Yuen KY, Zhang R, Jiang S, Structure-based discovery of Middle East respiratory syndrome corona virus fusion inhibitor. Nat Commun 2014, 5:3067
Lu R, Zhao X, Li J, et al. Genomic characterisation and epidemiology of 2019 novel coronavirus: implications for virus origins and receptor binding. Lancet 2020, 395:565-574.
Meyer K.C. Lung immunology and host defense. In: Bittar E.E. (eds) Pulmonary Biology in Health and Disease. 1993, Springer, New York, NY
Monforte V, Lopez-Sanchez A, Zurbano F, et al. Prophylaxis with nebulized liposomal amphotericin B for Aspergillus infection in lung transplant patients does not cause changes in the lipid content of pulmonary surfactant. J Heart Lung Transplant 2013, 32: 313-319.
Myers MA, Thomas DA, Straub L, et al. Pulmonary effects of chronic exposure to liposome aerosols in mice. Exp Lung Res 1993, 19:1-19.
Ong HX, Traini D, Young PM 2014, Chapter 7, Liposomes for Inhalation in: ISAM Textbook of Aerosol Medicine.
Osborne JL, Farmer R, Woodhouse KA, Self-assembled elastin-like polypeptide particles, Acta Biomaterialia 2008, 4, 49-57
Parthasarathy R, Gilbert B and Mehta K. Aerosol delivery of liposomal all-trans-retinoic acid to the lungs. Cancer Chemother Pharmacol 1999, 43:277-283.
Pellosi DS, d'Angelo I, Maiolino S, Mitidieri E, d'Emmanuele di Villa Bianca R, Sorrentino R, Quaglia F, Ungaro, In vitro/in vivo investigation on the potential of Pluronic® mixed micelles for pulmonary drug delivery, Eur J Pharm Biopharm 2018, 130:30-38
Perez-Soler R, Pilkiewicz F 2002, CA2456746A1 Method for treating lung cancer.
Rezazadeh M, Davatsaz Z, Emami J, Hasanzadeh F, Jahanian-Najafabadi A, Preparation and Characterization of Spray-Dried Inhalable Powders Containing Polymeric Micelles for Pulmonary Delivery of Paclitaxel in Lung Cancer, J Pharm Pharm Sci 2018, 21(1s), 200s-214s.
Riaz MK, Riaz MA, Zhang X, Lin C, Surface Functionalization and Targeting Strategies of Liposomes in Solid Tumor Therapy: A Review. Int J Mol Sci 2018, 19:195.
Ka Hong Wong 1, Xiaoyu Chen 1, Ge Zhang 1 ID , Aiping Lu 1,* and Zhijun Yang Rowlands RA, Cato MC, Waldschmidt HV, Bouley RA, Chen Q, Avramova L, Larsen SD, Tesmer JJG, White AD, Structure-Based Design of Selective, Covalent G Protein-Coupled Receptor Kinase 5 Inhibitors. ACS Med Chem Lett 2019,10:1628-1634.
Scheuch G, Meyer T, Sommerer K, Greindl A, Müllinger B, 2001, US7766012B2 Device for the controlled inhalation of therapeutic aerosols.
Shirley M, Amikacin Liposome Inhalation Suspension: A Review in Mycobacterium avium Complex Lung Disease. Drugs 2019, 79:555-562.
Singh J, Petter RC, Baillie TA, Whitty A, The resurgence of covalent drugs. Nat Rev Drug Discovery 2011, 10:307-317.
Skubitz KM and Anderson PM, Inhalational interleukin-2 liposomes for pulmonary metastases: a phase I clinical trial. Anticancer Drug 2000, 11:555-563.
Smyth HDC, Hickey A (Eds), Controlled Pulmonary Drug Delivery, Springer 2011.
Ten RM, Anderson PM, Zein NN, et al. Interleukin-2 liposomes for primary immune deficiency using the aerosol route. Int Immunopharmacol 2002, 2:333-344.
Torchilin VP and A. L. Klibanov AL, Immobilization of proteins on liposome surface, Enzyme Microb Technol 1981, 3:297-304.
Towler P, Staker B, Prasad SG, Menon S, Tang J, Parsons T, Ryan D, Fisher M, Williams D, Dales NA, Patane MA, and Pantoliano MW, ACE2 X-ray structures reveal a large hinge-bending motion important for inhibitor binding and catalysis. J Biol Chem 2004, 279: 17996-18007.
Verschraegen CF, Gilbert BE, Loyer E, et al. Clinical evaluation of the delivery and safety of aerosolized liposomal 9-nitro-20(S)-camptothecin in patients with advanced pulmonary malignancies. Clin Cancer Res 2004, 10:2319-2326.
Waldrep JC, Scherer PW, Hess GD, et al. Nebulized glucocortecoids in liposomes: aerosol characteristics and human dose estimates J Aerosol Med 1994, 7:135-145.
Weber C, Voigt M, Simon J, Danner AK, Frey H, Mailänder V, Helm M, Morsbach S, Landfester K, Functionalization of Liposomes with Hydrophilic Polymers Results in Macrophage Uptake Independent of the Protein Corona. Biomacromolecules 2019, 20:2989-2999.
Weers J. Lipid-based compositions of antiinfectives for treating pulmonary infections and methods of use thereof, 2006, US8226975B2.
Wysocki J, Ye M, Rodriguez E, et al. Targeting the Degradation of Angiotensin II with Recombinant ACE2: Prevention of Angiotensin II-dependent Hypertension. Hypertension 2010, 55:90-98.
Yan S, Sun H, Bu X and Wan G. An Evolutionary RGD Motif in the Spike Protein of SARS-CoV-2 may Serve as a Potential High Risk Factor for Virus Infection? Preprints 2020, 2020020447 (doi: 10.20944/preprints202002.0447.vl).
Zhang J, Leifer F, Rose S, et al. Amikacin Liposome Inhalation Suspension (ALIS) Penetrates Non-tuberculous Mycobacterial Biofilms and Enhances Amikacin Uptake Into Macrophages. Front Microbiol 2018, 9:915.
Zhang W, Song Y, Eldi P, Guo X, Hayball JD, Garg S, Albrecht H, Targeting prostate cancer cells with hybrid elastin-like polypeptide/liposome nanoparticles, Int J Nanomedicine 2018, 13, 293-305

## Claims

1. A vesicle, wherein the vesicle displays on its surface two or more different binding sites to one or more different docking domains on a pathogen.

2. The vesicle of claim 1, wherein at least one of the two or more binding sites on the surface of the vesicle comprise(s) a linker between the vesicle surface and the binding site, preferably wherein the linker is a polypeptide, a polysaccharide, or an organic polymer, preferably wherein the organic polymer is polyethylene glycol (PEG), preferably comprising 1 to 200 monomers.

3. The vesicle of any one of the preceding claims, wherein
(a) one of the two or more different binding sites to one or more different docking domains on the pathogen is an integrin or lectine; and/or
(b) one of the two or more different binding sites to one or more different docking domains on the pathogen is angiotensin converting enzyme 2 (ACE2); and/or
(c) one of the two or more different binding sites to one or more different docking domains on the pathogen is a chemical warhead for covalent binding to a reactive residue of a docking domain on the surface of the pathogen, preferably wherein the reactive residue is part of a phospholipid, polysaccharide and/or extracellular polypeptide, in particular an amino acid thereof like asparagine, threonine, lysine, tyrosine, cysteine, histidine, arginine.

4. The vesicle of claim 3(c), wherein the warhead and at least one other binding site of the two or more different binding sites to one or more different docking domains on the pathogen are arranged on the vesicle in a spatial proximity to allow covalent binding of the vesicle to at least one of the one or more different docking domains on the pathogen.

5. The vesicle of any one of the preceding claims, wherein the vesicle further displays
(a) a digestive enzyme, in particular an enzyme with polypeptide digestive activity, in particular a serine protease; and/or
(b) a macrophage binding site, in particular an activating domain, in particular an Fc domain of an IgG antibody.

6. The vesicle of any one of the preceding claims, wherein the vesicle has a diameter between 0.05 and 200 µm.

7. An aerosol comprising the vesicle of any one of the preceding claims.

8. The vesicle of any one of the preceding claims or the aerosol of claim 7 for use in medicine.

9. The vesicle of any one of the preceding claims or the aerosol of claim 7 or 8, which binds to a pathogen, preferably further comprising a payload, in particular an anti-sense oligonucleotide to block the pathogen DNA / RNA, a ribonuclease or trypsin or an antiviral drug or viricid.

10. The vesicle or aerosol of claim 9, wherein the pathogen is a virus.

11. The vesicle or aerosol of claim 10 for use in treating a viral infection, preferably wherein the virus is selected from the group consisting of Rhinovirus, RSV, HIV, Influenza virus, Parainfluenza virus, Metapneumovirus, Coronavirus, Enterovirus, Adenovirus, Bocavirus, Polyomavirus, Herpes simplex virus, and Cytomegalovirus.

12. The vesicle or aerosol of claim 9, wherein the pathogen is a bacterium or fungus.

13. The vesicle or aerosol of claim 11, wherein the Coronavirus is of the genus α-CoV, β-CoV, γ-CoV or δ-CoV, preferably wherein the Coronavirus is selected from the group consisting of Human coronavirus OC43 (HCoV-OC43), Human coronavirus HKU1 (HCoV- HKU1), Human coronavirus 229E (HCoV-229E), Human coronavirus NL63 (HCoV-NL63, New Haven coronavirus), Middle East respiratory syndrome-related coronavirus (MERS-CoV or "novel coronavirus 2012"), Severe acute respiratory syndrome coronavirus (SARS-CoV or "SARS-classic"), and Severe acute respiratory syndrome coronavirus 2 (SARS-CoV-2 or "novel coronavirus 2019").

14. A solid surface coated with the vesicle of any one of claims 1 to 6, preferably wherein the solid surface is a mask or an air filter.

15. A method for diagnosing a disease, the method comprising the use of the vesicle of any one claims 1 to 6 or of the aerosol of claim 7, wherein the vesicle or aerosol comprises a detectable label.
